(19)

Europäisches Patentamt
European Patent Office
Office européen des brevets

(11) **EP 4 385 412 A1**

(12) **EUROPEAN PATENT APPLICATION**
published in accordance with Art. 153(4) EPC

(43) Date of publication:
**19.06.2024 Bulletin 2024/25**

(21) Application number: **22869583.9**

(22) Date of filing: **09.03.2022**

(51) International Patent Classification (IPC):
*A61B 5/31* $^{(2021.01)}$     *A61B 5/369* $^{(2021.01)}$

(52) Cooperative Patent Classification (CPC):
**A61B 5/31; A61B 5/369**

(86) International application number:
**PCT/JP2022/010415**

(87) International publication number:
**WO 2023/042431 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.09.2021   JP 2021151823**

(71) Applicant: **Sony Group Corporation
Tokyo 108-0075 (JP)**

(72) Inventors:
• **KITAKAMI, Yukinojo
Tokyo 108-0075 (JP)**
• **ISHIKAWA, Takanori
Tokyo 108-0075 (JP)**

(74) Representative: **D Young & Co LLP
3 Noble Street
London EC2V 7BQ (GB)**

(54) **MEASUREMENT DEVICE AND MEASUREMENT METHOD**

(57)     A measurement device of the present disclosure includes: a sensor configured to generate a first biological signal that is based on first biological information; a processor configured to generate a second biological signal by performing a noise reduction process on the basis of the first biological signal; and a storage configured to store a processing result of the noise reduction process, The processor is configured to perform the noise reduction process using the processing result stored in the storage on the basis of the first biological signal.

[ FIG. 7 ]

# Description

[Technical Field]

The present disclosure relates to a measurement device that measures a biological signal that is based on biological information and a measurement method to measure a biological signal that is based on a biological state.

[Background Art]

In the field of medical services, a biological signal that is based on biological information regarding a living body such as a human body is often detected. For example, PTL 1 discloses a device that reduces a noise component contained in a biological signal obtained from a sensor and estimates an activity within a brain on the basis of the biological signal with the reduced noise component.

[Citation List]

[Patent Literature]

PTL 1: Japanese Unexamined Patent Application Publication No. 2001-000407

[Summary of the Invention]

As seen from the above, as for a measurement device, reducing a noise component is desired and, accordingly, the noise component is expected to be effectively reduced.

It is desirable to provide a measurement device and a measurement method that enable effectively reducing a noise component.

A measurement device of an embodiment of the present disclosure includes a sensor, a processor, and a storage. The sensor is configured to generate a first biological signal that is based on first biological information. The processor is configured to generate a second biological signal by performing a noise reduction process on the basis of the first biological signal. The storage is configured to store a processing result of the noise reduction process. The processor is configured to perform the noise reduction process using the processing result stored in the storage on the basis of the first biological signal.

A measurement method of an embodiment of the present disclosure includes: generating a first biological signal that is based on first biological information; generating a second biological signal by performing a noise reduction process on the basis of the first biological signal; and storing a processing result of the noise reduction process. The noise reduction process is performed using the stored processing result on the basis of the first biological signal.

In the measurement device and the measurement method of the embodiments of the present disclosure, a first biological signal is generated according to first biological information and a second biological signal is generated by performing a noise reduction process on the basis of the first biological signal. Moreover, the processing result of the noise reduction process is stored. The noise reduction process is performed using the stored processing result on the basis of the first biological signal.

[Brief Description of Drawing]

[FIG. 1] FIG. 1 is a block diagram illustrating a configuration example of a measurement device according to an embodiment of the present disclosure.

[FIG. 2] FIG. 2 is a diagram of assistance in explaining a configuration example of a sensor illustrated in FIG. 1.

[FIG. 3A] FIG. 3A is a waveform diagram illustrating an example of a potential signal illustrated in FIG. 1.

[FIG. 3B] FIG. 3B is a waveform diagram illustrating an example of a potential signal illustrated in FIG. 1.

[FIG. 4] FIG. 4 is a block diagram illustrating a configuration example of a processor illustrated in FIG. 1.

[FIG. 5] FIG. 5 is a diagram of assistance in explaining an example of a matrix generated by a signal separation transformer illustrated in FIG. 4.

[FIG. 6] FIG. 6 is a diagram of assistance in explaining an operation example of the matrix transformer illustrated in FIG. 4.

[FIG. 7] FIG. 7 is a flowchart illustrating an operation example of the measurement device illustrated in FIG. 1.

[FIG. 8] FIG. 8 is a diagram of assistance in explaining an example of a matrix generated by the signal separation transformer according to a reference example.

[FIG. 9] FIG. 9 is a diagram of assistance in explaining an operation example of the matrix transformer according to a reference example.

[FIG. 10] FIG. 10 is a diagram of assistance in explaining another operation example of the matrix transformer illustrated in FIG. 4.

[FIG. 11] FIG. 11 is a block diagram illustrating a configuration example of a measurement device according to a modification example.

[FIG. 12] FIG. 12 is a block diagram illustrating a configuration example of the processor illustrated in FIG. 11.

[Modes for Carrying Out the Invention]

A detailed description will be made below on an embodiment of the present disclosure with reference to the drawings.

<Embodiment>

[Configuration Example]

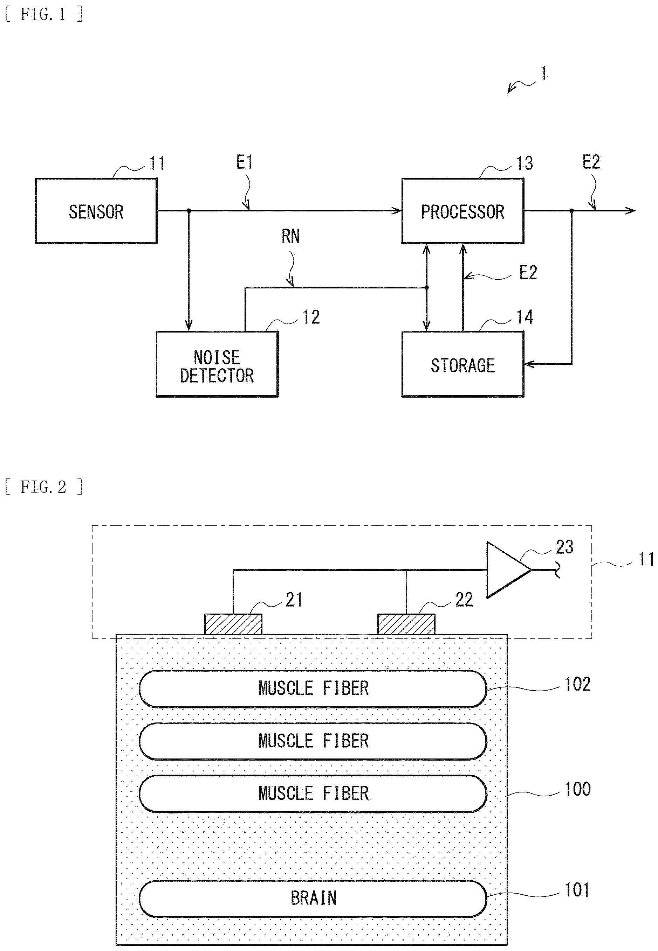

FIG. 1 illustrates a configuration example of a measurement device (a measurement device 1) according to an embodiment. The measurement device 1 is a brain wave potential measurement device that measure a brain wave of a huma body. The measurement device 1 includes a sensor 11, a noise detector 12, a processor 13, and a storage 14. The noise detector 12, the processor 13, and the storage 14 include, for example, a processor, a memory, and the like.

The sensor 11 is configured to be brought into contact with, for example, a head, an ear, or the like of a huma body to generate a potential signal E1 according to a brain wave of the human body. The potential signal E1 is a signal (a potential signal E1(t)) changeable with time t. The measurement device 1 is a single-channel measurement device that performs a process on the basis of such a single potential signal E1.

FIG. 2 illustrates a configuration example of the sensor 11. The sensor 11 includes, for example, electrodes 21, 22 and an amplifier 23. The electrodes 21, 22 are configured to be able to be brought into contact with a head 100 of a measurement target, or human body. The amplifier 23 is configured to be able to amplify signals obtained by the electrodes 21, 22. The sensor 11 generates the potential signal E1 according to a neural activity of a brain 101 in the head 100.

A muscle fiber 102 is present near a surface of the head 100. The muscle fiber 102 performs a contraction motion and a relaxation motion on the basis of an action potential generated by a motor cell. The potential signal E1 to be generated by the sensor 11 would contain a signal component according to such an action potential of the muscle fiber 102 in addition to a signal component according to the neural activity of the brain 101. As the measurement device 1 measures a brain wave, the signal component according to such an action potential of the muscle fiber 102 is noise (hereinafter, also referred to as myoelectric noise).

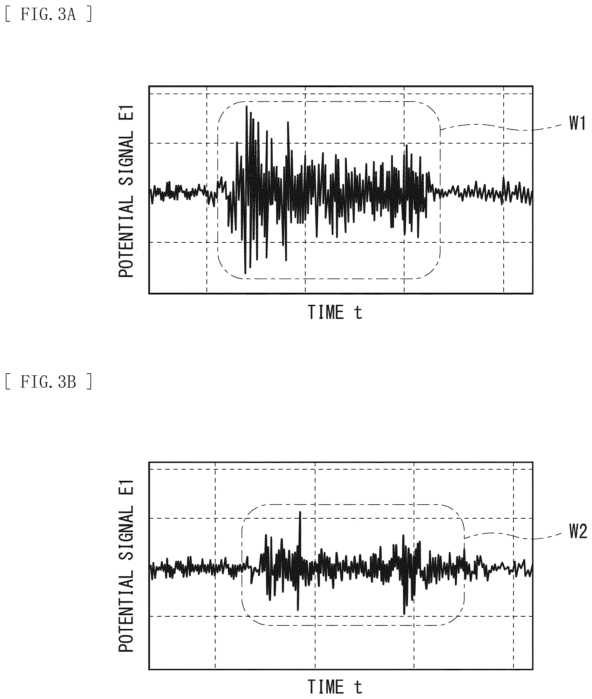

FIGs. 3A, 3B illustrate examples of the myoelectric noise in the potential signal E1.

For example, in a case where a person performs an action with a motion of a skeleton, such as a chewing action, large action potentials are produced in a plurality of muscle fibers 102. As a result, a signal component with a large amplitude intensity is produced in the potential signal E1 as illustrated at a portion W1 in FIG. 3A. In other words, in the potential signal E1, the signal component with the large amplitude intensity according to the action potentials of the muscle fibers 102 is superimposed on a continuously appearing signal component of a brain wave only for a short time.

Meanwhile, for example, in a case where a person changes his/her facial expression, such as grimaces, small action potentials are produced in a few of the muscle fibers 102 closer to the surface of the head 100. As a result, a signal component with a small amplitude intensity is produced in the potential signal E1 as illustrated at a portion W2 in FIG. 3B. In other words, in the potential signal E1, the signal component with the small amplitude intensity according to the action potentials of the muscle fibers 102 is superimposed on a continuously appearing signal component of a brain wave only for a short time.

As seen from the above, in the potential signal E1, myoelectric noises with a variety of amplitude intensities would be superimposed. In addition, although a myoelectric noise is produced for a short time in the examples in FIGs. 3A, 3B, a myoelectric noise would be intermittently produced for a long time. The measurement device 1 is configured to generate a potential signal E2 by reducing such a variety of myoelectric noises from the single potential signal E1.

The noise detector 12 is configured to calculate a degree of inclusion of a myoelectric noise in the potential signal E1. In this example, the noise detector 12 calculates a time ratio RN of a period where a myoelectric noise is contained in the potential signal E1 to an entire period of the potential signal E1. Specifically, the noise detector 12 monitors the potential signal E1 throughout a period with a time length T and calculates the time ratio RN of the period where the myoelectric noise is produced in the potential signal E1. Moreover, the noise detector 12 supplies information regarding the time ratio RN to the processor 13 and the storage 14.

The processor 13 (FIG. 1) is configured to generate the potential signal E2 by performing a noise reduction process on the basis of the potential signal E1 supplied from the sensor 11. In a case where the time ratio RN of the noise obtained by the noise detector 12 is equal to or less than a predetermined value TH, the processor 13 performs the noise reduction process on the basis of the potential signal E1. The predetermined value TH may be, for example, 20%. Meanwhile, in a case where the time ratio RN of the noise is greater than the predetermined value TH, the processor 13 performs the noise reduction process using the potential signal E2 supplied from the storage 14 on the basis of the potential signal E1. As seen from the above, in a case where the amount of myoelectric noise contained in the potential signal E1 is large, the processor 13 performs the noise reduction process using the potential signal E2 supplied from the storage 14.

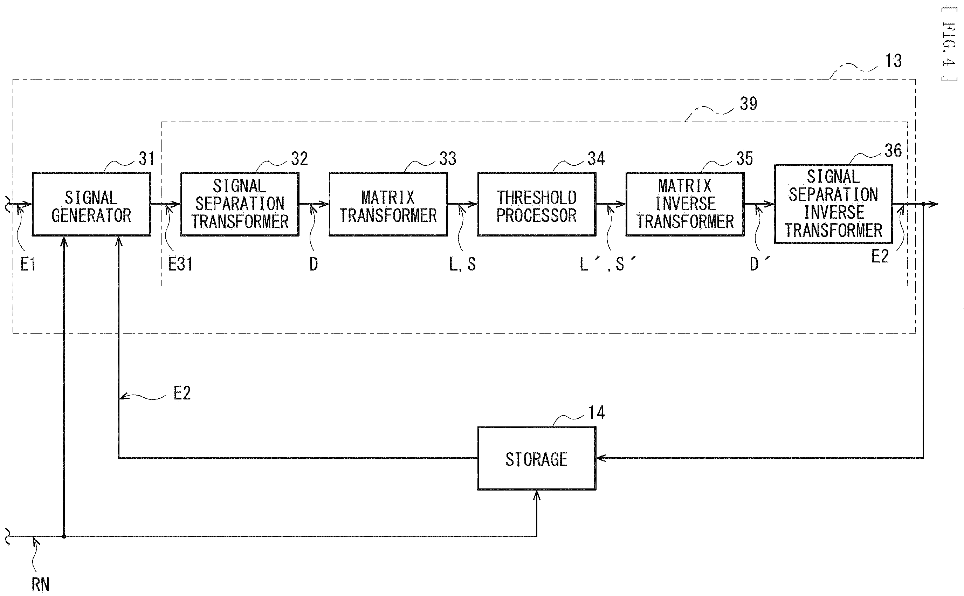

FIG. 4 illustrates a configuration example of the processor 13. FIG. 4 also illustrates the storage 14 for the convenience of explanation. The processor 13 includes a signal generator 31 and a noise reduction processor 39. The noise reduction processor 39 includes a signal separation transformer 32, a matrix transformer 33, a threshold processor 34, a matrix inverse transformer 35, and a signal separation inverse transformer 36.

The signal generator 31 is configured to generate a potential signal E31 to be inputted to the noise reduction processor 39 on the basis of the potential signal E1. Specifically, in a case where the time ratio RN of the noise obtained by the noise detector 12 is equal to or less than the predetermined value TH, the signal generator 31 directly outputs the potential signal E1 as the potential signal E31. Meanwhile, in a case where the time ratio RN of the noise obtained by the noise detector 12 is greater than the predetermined value TH, the signal generator 31 generates the potential signal E31 by combining the potential signal E1 with the potential signal E2 supplied from the storage 14 on a time axis.

The signal separation transformer 32 is configured to generate a matrix D by separating the potential signal E31 into signals with a plurality of frequency components. Specifically, the signal separation transformer 32 generates absolute values d (t, f) of a spectrogram by performing the short-time Fourier transform on the basis of the potential signal E31 during the period of the time length T (for example, one minute). Here, t is time and f is frequency. Moreover, the signal separation transformer 32 generates the matrix D including the absolute values d(t, f) as matrix elements.

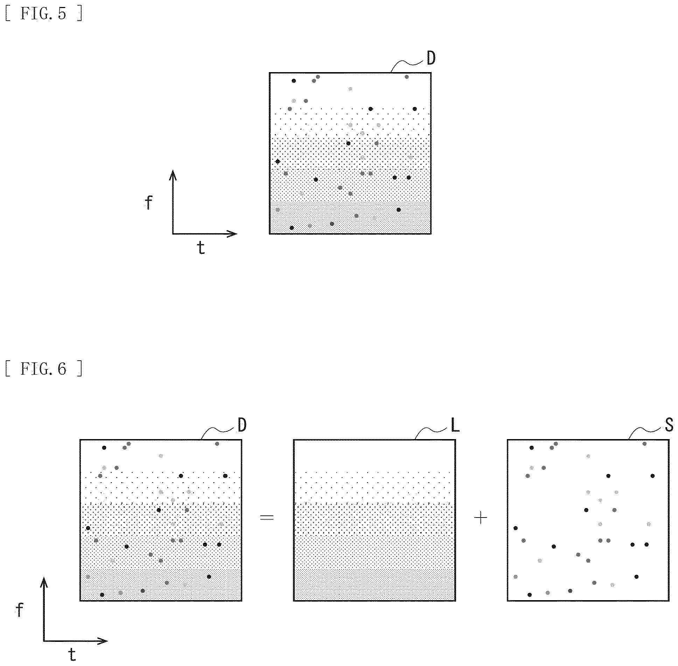

FIG. 5 schematically illustrates the matrix D. The horizontal axis represents the time t and the vertical axis represents frequency f. In the matrix D, the matrix element, or absolute values d(t, f) of the spectrogram, are arranged. In FIG. 5, the magnitudes of the values of the matrix elements are indicated by hatching and dots.

The matrix D contains the characteristics of a brain wave and the characteristics of a myoelectric noise. Since the brain wave is continuously produced in a time-axis direction, a component of the brain wave is less changeable in the time-axis direction in the matrix D. In addition, the component of the brain wave becomes large with a reduction in frequency and becomes small with an increase in frequency. As seen from the above, the component of the brain wave has commonality in the time-axis direction and a frequency-axis direction. The matrix D thus has characteristics as indicated by the hatching in FIG. 5. A light hatching indicates that the values of the matrix elements are small and a dark hatching indicates that the values of the matrix elements are large. In contrast, a myoelectric noise is discretely produced in, for example, the time-axis direction and the frequency-axis direction. In other words, a component of the myoelectric noise has sparsity in the time-axis direction and the frequency-axis direction. The matrix D thus has characteristics as indicated by the dots in FIG. 5. Light dots indicate that the values of the matrix elements are small and dark dots indicate that the values of the matrix elements are large.

The signal separation transformer 32 generates such a matrix D. Moreover, the signal separation transformer 32 supplies the generated matrix D to the matrix transformer 33.

The matrix transformer 33 (FIG. 4) is configured to generate a low-rank matrix L and a sparse matrix S by performing a matrix separation process on the basis of the matrix D. The matrix D is represented by the sum of the low-rank matrix L and the sparse matrix S as indicated below.

$$D = L + S$$

The low-rank matrix L includes the matrix elements less changeable in the time-axis direction. The low-rank matrix L is a matrix having less eigenvalues calculated by a principal component analysis. The sparse matrix S includes the matrix elements that are discrete in the time-axis direction and the frequency-axis direction. The sparse matrix S is a matrix having less matrix elements values of which are not zero. The matrix transformer 33 separates the matrix D into the low-rank matrix L and the sparse matrix S to cause a value below to reach the minimum.

$$\|L\|_* + \lambda \|\psi(S)\|_1$$

Here, "$\|L\|_*$" is a nuclear norm of the low-rank matrix L. "$\|L\|_*$" is the sum of singular values of the low-rank matrix L. $\psi$ in this example is transform that generates a matrix by calculating a difference in the frequency-axis direction between a plurality of matrix elements in the sparse matrix S. The transform $\psi$ emphasizes a sparse component in the sparse matrix S. "$\|\psi(S)\|_1$" is a L1 norm of the matrix generated by the transform $\psi$. "$\|\psi(S)\|_1$" is the sum of the absolute values of the elements in the matrix generated by the transform $\psi$. $\lambda$ is an adjustment parameter for the matrix separation process and is a scalar quantity of a predetermined value in a range from "0" to "1."

FIG. 6 schematically illustrates an example of the matrix separation process of the matrix transformer 33. The matrix transformer 33 generates the low-rank matrix L and the sparse matrix S by performing the matrix separation process on the basis of the matrix D. The low-rank matrix L has the characteristics indicated by the hatching in FIG. 5 and mainly contains a component of a brain wave. Meanwhile, the sparse matrix S has the characteristics indicated by dots in FIG. 5 and mainly contains a component of a myoelectric noise. The adjustment parameter $\lambda$ is thus adjustable to cause the component of the brain wave and the component of the myoelectric noise to be separated into the low-rank matrix L and the sparse matrix S, respectively.

As seen from the above, the matrix transformer 33 generates the low-rank matrix L mainly containing the component of the brain wave and the sparse matrix S mainly containing the component of the myoelectric noise by performing the matrix separation process.

The threshold processor 34 (FIG. 4) is configured to generate a low-rank matrix L' and a sparse matrix S' on the basis of the low-rank matrix L and the sparse matrix S generated by the matrix transformer 33. Specifically, the threshold processor 34 outputs the low-rank matrix L as the low-rank matrix L' and generates the sparse matrix S' by multiplying the sparse matrix S by a zero matrix. The low-rank matrix L' and the sparse matrix S' may be represented as the following expressions.

$$L' = L$$

$$S' = 0$$

The matrix inverse transformer 35 is configured to calculate a matrix D' by calculating the sum of the low-rank matrix L' and the sparse matrix S'. In this example, since the sparse matrix S' is the zero matrix, the matrix D' is equal to the low-rank matrix L'.

The signal separation inverse transformer 36 is configured to generate the potential signal E2 by performing the inverse transform of the short-time Fourier transform performed by the signal separation transformer 32 on the basis of the matrix D'. The potential signal E2 is a signal (a potential signal E2(t)) changeable with the time t during a period of the time length T (for example, one minute).

By virtue of this configuration, the noise reduction processor 39 generates the potential signal E2 on the basis of, out of the low-rank matrix L and the sparse matrix S obtained through the matrix separation process, the low-rank matrix L. As seen from the above, the noise reduction processor 39 generates the potential signal E2 by reducing the component of the myoelectric noise contained in the potential signal E1.

The storage 14 (FIG. 2) is configured to store the potential signal E2 generated by the processor 13. Specifically, in a case where the time ratio RN of the noise obtained by the noise detector 12 is equal to or less than the predetermined value TH, the storage 14 stores the potential signal E2 generated by the processor 13. Moreover, in a case where the time ratio RN of the noise is greater than the predetermined value TH, the storage 14 supplies the stored potential signal E2 to the processor 13.

Here, the sensor 11 corresponds to a specific example of a "sensor" of the present disclosure. The potential signal E1 corresponds to a specific example of a "first biological signal" of the present disclosure. The processor 13 corresponds to a specific example of a "processor" of the present disclosure. The matrix D corresponds to a specific example of a "first matrix" of the present disclosure. The matrix D corresponds to a specific example of a "first matrix" of the present disclosure. The low-rank matrix L corresponds to a specific example of a "second matrix" of the present disclosure. The sparse matrix S corresponds to a specific example of a "third matrix" of the present disclosure. The potential signal E2 corresponds to a specific example of a "second biological signal" of the present disclosure. The storage 14 corresponds to a specific example of a "storage" of the present disclosure. The noise detector 12 corresponds to a specific example of a "noise detector" of the present disclosure.

[Operations and Workings]

Subsequently, description will be made on operations and workings of the measurement device 1 according to the present embodiment.

(Summary of Overall Operation)

First, the summary of an overall operation of the measurement device 1 will be described with reference to FIG. 1. The sensor 11 is brought into contact with the head, ear, or the like of a huma body and generates a potential signal E1 according to a brain wave of the human body. The noise detector 12 calculates the degree of inclusion of a myoelectric noise in the potential signal E1. Specifically, the noise detector 12 calculates a time ratio RN of a period where a myoelectric noise is contained in the potential signal E1 to an entire period of the potential signal E1. The processor 13 generates a potential signal E2 by performing a noise reduction process on the basis of the potential signal E1 supplied from the sensor 11. Specifically, in a case where the time ratio RN of the noise obtained by the noise detector 12 is equal to or less than a predetermined value TH, the processor 13 performs the noise reduction process on the basis of the potential signal E1. Meanwhile, in a case where the time ratio RN of the noise is greater than the predetermined value TH, the processor 13 performs the noise reduction process using the potential signal E2 supplied from the storage 14 on the basis of the potential signal E1. The storage 14 stores the potential signal E2 generated by the processor 13. Specifically, in a case where the time ratio RN of the noise obtained by the noise detector 12 is equal to or less than the predetermined value TH, the storage 14 stores the potential signal E2 generated by the processor 13. Moreover, in a case where the time ratio RN of the noise is greater than the predetermined value TH, the storage 14 supplies the stored potential signal E2 to the signal generator 31 of the processor 13.

(Detailed Operations)

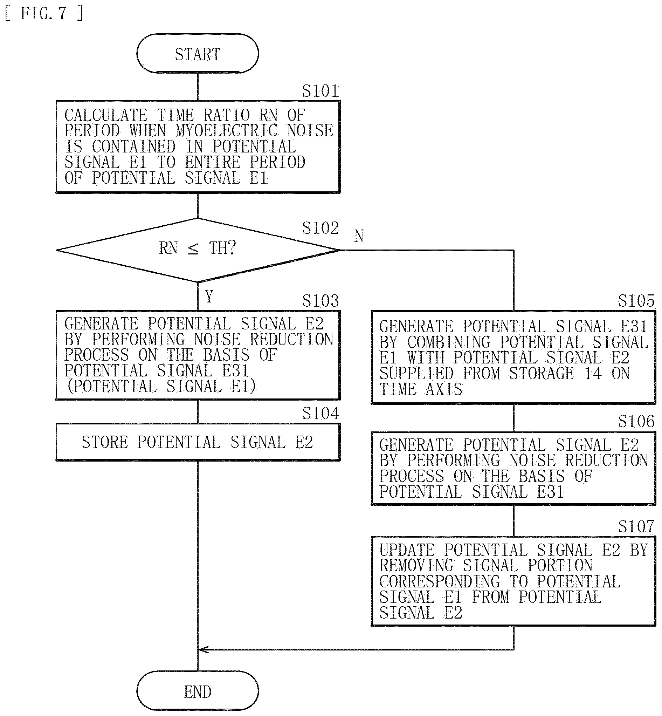

FIG. 7 illustrates an operation example of the measurement device 1. The measurement device 1 performs the following process every time when acquiring a potential signal E1 during the period with the time length T.

First, the noise detector 12 calculates a time ratio RN of a period when a myoelectric noise is contained in the potential signal E1 to the entire period of the potential signal E1 (Step S101).

Next, the measurement device 1 checks whether or not the time ratio RN of the noise is equal to or less than the predetermined value TH (R ≤ TH) (Step S102). The predetermined value TH may be, for example, 20%.

In Step S102, in a case where the time ratio RN of the noise is equal to or less than the predetermined value TH (in Step S102, "Y"), the signal generator 31 of the processor 13 directly supplies the potential signal E1 as a potential signal E31 to the noise reduction processor 39 and the noise reduction processor 39 generates a potential signal E2 by performing the noise reduction process on the basis of the potential signal E31 (Step S103).

Specifically, in the noise reduction processor 39, the signal separation transformer 32 first generates a matrix D as illustrated in FIG. 5 by separating the potential signal E31 into signals with a plurality of frequency components. The matrix transformer 33 generates a low-rank matrix L and a sparse matrix S by applying the matrix separation process to the matrix D as illustrated in FIG. 6. The low-rank matrix L mainly contains a component of a brain wave and the sparse matrix S mainly contains a myoelectric noise. The threshold processor 34 outputs the low-rank matrix L as a low-rank matrix L' and generates a sparse matrix S' by multiplying the sparse matrix S by the zero matrix. The matrix inverse transformer 35 calculates a matrix D' by calculating the sum of the low-rank matrix L' and the sparse matrix S'. The signal separation inverse transformer 36 generates a potential signal E2 by performing the inverse transform of the short-time Fourier transform performed by the signal separation transformer 32 on the basis of the matrix D'. The noise reduction processor 39 generates the potential signal E2 by reducing the myoelectric noise contained in the potential signal E1 on the basis of the potential signal E1 in this manner.

Next, the storage 14 stores the potential signal E2 generated in Step S103 (Step S104). The potential signal E2 stored in the storage 14 is to be used for the next and subsequent processes.

In Step S102, in a case where the time ratio RN of the noise is greater than the predetermined value TH (in Step S102, "N"), the signal generator 31 of the processor 13 generates a potential signal E31 by combining the potential signal E1 with the potential signal E2 supplied from the storage 14 on a time axis. (Step S105).

Next, the noise reduction processor 39 of the processor 13 generates a potential signal E2 by performing a noise reduction process on the basis of the potential signal E31 (Step S106). The noise reduction process in Step S106 is the same as the noise reduction process in Step S103 except that the potential signal E31 to be inputted contains the potential signal E1 and the electric signal E2.

Moreover, the signal separation inverse transformer 36 of the noise reduction processor 39 updates the potential signal E2 by removing a signal portion corresponding to the potential signal E1 from the potential signal E2 (Step S107).

The flow is then terminated.

As seen from the above, in the measurement device 1, in a case where the time ratio RN of the noise is greater than the predetermined value TH (in Step S102, "N"), the potential signal E31 is generated by combining the potential signal E1 with the potential signal E2 supplied from the storage 14 on the time axis (Step S105) and the potential signal E2 is generated by performing the noise reduction process on the basis of the potential signal E31 (Step S106). Thus, in a case where a myoelectric noise is continuously produced for a long time, the measurement device 1 is able to effectively reduce the myoelectric noise.

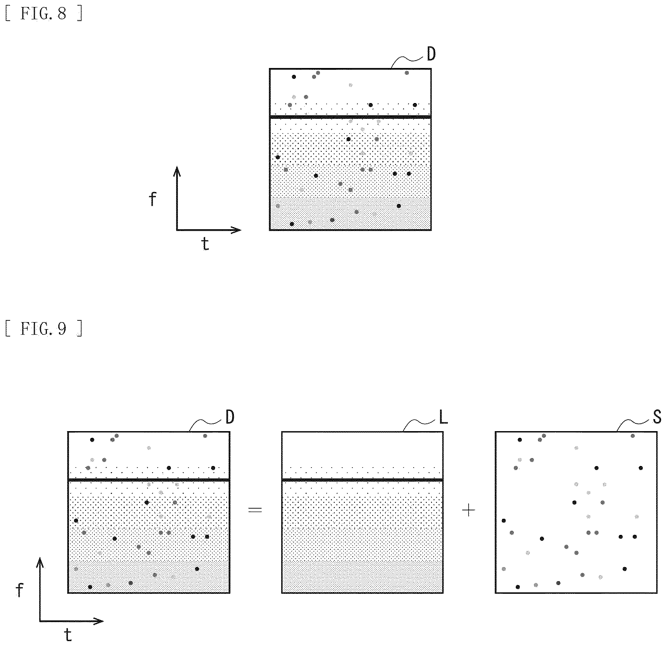

In other words, if a myoelectric noise is continuously produced for a long time, it is likely that the myoelectric noise fails to be reduced by performing the noise reduction process on the basis of only the potential signal E1 as in Step S103. In other words, in a case where a myoelectric noise is continuously produced for a long time as above, the matrix D would contain characteristics indicated by a line in addition to characteristics indicated by hatching and dots as illustrated in FIG. 8. The line is formed by alignment of the matrix elements having large values in the time-axis direction. The matrix transformer 33 generates a low-rank matrix L and a sparse matrix S by performing the matrix separation process on the basis of such a matrix D as illustrated in FIG. 9. The low-rank matrix L has the characteristics indicated by the hatching and the line in FIG. 8. In other words, the low-rank matrix L also contains a component of the continuously produced myoelectric noise in addition to the component of the brain wave. As a result, it is not possible to effectively reduce the myoelectric noise in this example.

Accordingly, in a case where a myoelectric noise is continuously produced for a long time, the measurement device 1 generates a potential signal E31 by combining the potential signal E1 with the potential signal E2 supplied from the storage 14 on the time axis (Step S105). Moreover, the measurement device 1 generates a potential signal E2 by performing the noise reduction process on the basis of the potential signal E31 (Step S106).

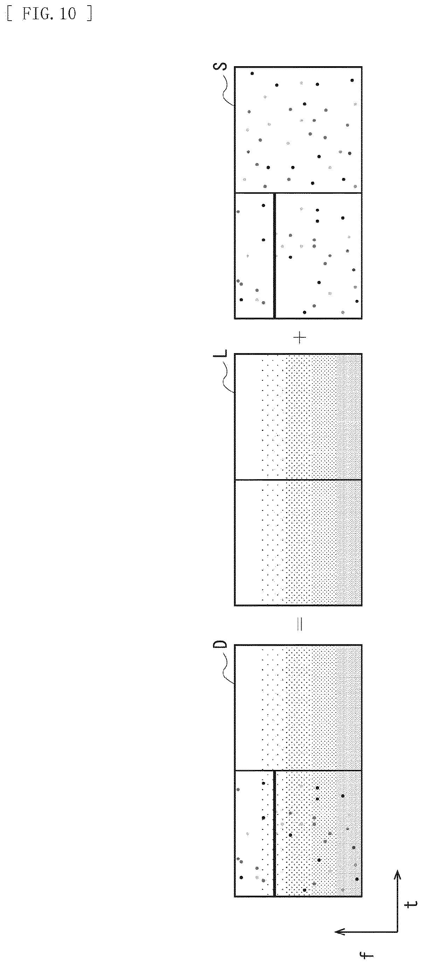

FIG. 10 schematically illustrates an example of the matrix separation process in Step S106. The matrix D is generated on the basis of the combined potential signal E31, which causes the matrix D to have a size that is twice as large as the matrix D illustrated in FIG. 9 in the time-axis direction. A left half of the matrix D corresponds to the potential signal E1 and a right half thereof corresponds to the potential signal E2 supplied from the storage 14. The matrix transformer 33 generates a low-rank matrix L and a sparse matrix S by performing the matrix separation process on the basis of such a matrix D. The low-rank matrix L likewise has a size that is twice as large as the low-rank matrix L illustrated in FIG. 9 in the time-axis direction and the sparse matrix S likewise has a size that is twice as large as the sparse matrix S illustrated in FIG. 9 in the time-axis direction. As illustrated in FIG. 10, the low-rank matrix L has the characteristics indicated by the hatching in FIG. 8 and mainly contains the component of the brain wave. Meanwhile, the sparse matrix S has the characteristics indicated by the dots and the line in FIG. 8 and mainly contains the component of the myoelectric noise. The component of the continuously produced myoelectric noise is contained in the sparse matrix S. In other words, the low-rank matrix L contains the component of the continuously produced myoelectric noise in the example in FIG. 9, whereas the sparse matrix S contains the component of the continuously produced myoelectric noise in the example in FIG. 10. This makes it possible for the measurement device 1 to effectively reduce the noise component.

As seen from the above, the measurement device 1 includes the sensor 11 that generates a first biological signal (a potential signal E1) according to information regarding a brain wave, the processor 13 that generates a second biological signal (a potential signal E2) by performing the noise reduction process on the basis of the first biological signal (the potential signal E1), and the storage 14 that stores a processing result (the potential signal E2) of the noise reduction process. Moreover, the processor 13 performs the noise reduction process using the processing result (the potential signal E2) stored in the storage 14 on the basis of the first biological signal (the potential signal E1). Thus, for example, in a case where a myoelectric noise is continuously produced for a long time, the measurement device 1 is able to reduce the myoelectric noise as illustrated in FIG. 10. As a result, the measurement device 1 is able to effectively reduce the noise component.

In addition, the measurement device 1 includes the noise detector 12 that calculates the noise degree (the time ratio RN of the noise) of inclusion of noise that is based on activity information regarding the muscle fiber in the first biological signal (the potential signal E1). Moreover, in a case where the noise degree is greater than the predetermined value, the processor 13 performs the noise reduction process using the processing result (the potential signal E2) stored in the storage 14 on the basis of the first biological signal (the potential signal E1). In addition, in a case where the noise degree is smaller than the predetermined value, the processor 13 performs the noise reduction process on the basis of the first biological signal (the potential signal E1). This makes it possible for the measurement device 1 to cause the second biological signal (the potential signal E2) obtained in a case where the amount of noise is small to be stored in the storage 14 and perform, in a case where the amount of noise is large, the noise reduction process on the basis of the potential signal E1 and the potential signal E2 stored in the storage 14 as illustrated in FIG. 10. As a result, the measurement device 1 is able to effectively reduce the noise component.

In addition, in the measurement device 1, the noise reduction process includes generating a first matrix (a matrix D) by separating the first biological signal (the potential signal E1) into a plurality of signals, performing the matrix separation process to separate the first matrix (the matrix D) into a second matrix (a low-rank matrix L) having a component less changeable in the time-axis direction and a third matrix (a sparse matrix S) having a component that is discrete in the time-axis direction, and generating a second biological signal (a potential signal E2) on the basis of the second matrix (the low-rank matrix L). During the matrix separation process, the processor 13 generates a fourth matrix ($\psi$(S)) by performing an emphasis process to emphasize the third matrix (the sparse matrix S) and separates the first matrix (the matrix D) into the second matrix (the low-rank matrix L) and the third matrix (the sparse matrix S) to cause the second matrix (the low-rank matrix L) and the fourth matrix ($\psi$(S)) to satisfy a predetermined condition. Thus, in the measurement device 1, for example, even in a case where the amplitude intensity of the myoelectric noise is close to the amplitude intensity of the brain wave as illustrated in FIG. 3B, the signal component of the myoelectric noise is emphasized, which makes it possible to reduce the myoelectric noise. As a result, the measurement device 1 is able to effectively reduce the noise component.

This makes it possible to reduce the number of channels in the measurement device 1; therefore, the number of sensors 11 to be coupled to a user is allowed to be reduced to enhance comfortability. In the measurement device 1, it is possible to generate, irrespective of such a small number of channels, a potential signal E2 with a reduced number of noise components, allowing the accuracy of measurement of the brain wave to be enhanced.

[Effects]

As described above, in the present embodiment, a sensor that generates a first biological signal that is based on brain wave information, a processor that generates a second biological signal by performing a noise reduction process on the basis of the first biological signal, and a storage that stores a processing result of the noise reduction process are provided. Moreover, the processor performs the noise reduction process using the processing result stored in the storage on the basis of the first biological signal. This makes it possible to effectively reduce a noise component.

In the present embodiment, a noise detector that calculates a noise degree of inclusion of noise that is based on activity information regarding a muscle fiber in the first biological signal is provided. Moreover, in a case where the noise degree is greater than a predetermined value, the processor performs the noise reduction process using the processing result stored in the storage on the basis of the first biological signal. In addition, in a case where the noise degree is smaller than the predetermined value, the processor performs the noise reduction process on the basis of the first biological signal. This makes it possible to effectively reduce a noise component.

In addition, in the measurement device 1, the noise reduction process includes generating a first matrix by separating the first biological signal into a plurality of signals, performing the matrix separation process to separate the first matrix into a second matrix having a component less changeable in a time-axis direction and a third matrix having a component that is discrete in the time-axis direction, and generating a second biological signal on the basis of the second matrix. During the matrix separation process, the processor generates a fourth matrix by performing an emphasis process to emphasize the third matrix and separates the first matrix into the second matrix and the third matrix to cause the second matrix and the fourth matrix to satisfy a predetermined condition. This makes it possible to effectively reduce a noise component.

[Modification Example 1]

In the above-described embodiment, the measurement device 1 is configured as a single-channel measurement device that performs the noise reduction process on the basis of the single potential signal E1; however, this is not limiting. Alternatively, the measurement device 1 may perform the noise reduction process on the basis of a plurality of potential signals E1. In this case, for example, a plurality of circuit groups each including the sensor 11, the noise detector 12, the processor 13, and the storage 14 illustrated in FIG. 1 may be provided. In addition, for example, a plurality of sensors 11 may be provided to allow the processor 13 to generate, for example, a three-dimensional matrix D on the basis of a plurality of potential signals E1 generated by the plurality of sensors 11 and perform the process on the basis of the matrix D.

[Modification Example 2]

In the above-described embodiment, the processor of the measurement device 1 performs all the processes to generate the potential signal E2 by performing the noise reduction process on the basis of the potential signal E1; however, this is not limiting. Alternatively, for example, a part of these processes may be performed by an arithmetic device other than the measurement device. Specifically, for example, a personal computer, a smartphone, a cloud network, or the like is able to perform a part of these processes.

[Modification Example 3]

In the above-described embodiment, the signal separation transformer 32 generates the matrix D by performing the short-time Fourier transform on the basis of the potential signal E31; however, this is not limiting. Alternatively, the matrix D may be generated by, for example, performing wavelet transform, discrete cosine transform, empirical mode decomposition, or the like on the basis of the potential signal E31. In addition, for example, in a case where the process is to be performed on the basis of a plurality of potential signals E1, the signal separation transformer 32 is able to generate the matrix D by performing, for example, principal component analysis, canonical correlation analysis, or independent component analysis.

[Modification Example 4]

In the above-described embodiment, during the transform $\psi$, the matrix transformer 33 calculates a difference in the frequency-axis direction between a plurality of matrix elements of the matrix S; however, this is not limiting. Alternatively, for example, the matrix transformer 33 may calculate a difference in the time-axis direction between the plurality of matrix elements or may calculate a difference in the time-axis direction as well as calculate a difference in the frequency-axis direction between the plurality of matrix elements. In addition, in this example, the matrix transformer 33 calculates a difference during the transform $\psi$; however, this is not limiting and wavelet transform or discrete cosine transform may be performed. In this case, the transform $\psi$ is also able to emphasize a sparse component in the matrix.

[Modification Example 5]

In the above-described embodiment, a myoelectric noise is reduced; however, this is not limiting. Alternatively, for example, an ophthalmic potential noise attributed to eye movements, a cardiac potential noise attributed to heartbeats, a sweating noise, or a contact noise resulting from a change in a contact state between the electrodes 21, 22 and the skin may be reduced. The signal separation transformer 32 may select, as a process to be applied, one of, for example, short-time Fourier transform, wavelet transform, discrete cosine transform, and empirical mode decomposition in accordance with the types of noises to reduce. Likewise, the matrix transformer 33 may select, as a process to be applied during the transform $\psi$, one of a process to calculate a difference, wavelet transform, and discrete cosine transform in accordance with the types of noises to reduce.

[Modification Example 6]

In the above-described embodiment, the threshold processor 34 directly outputs the low-rank matrix L as the low-rank matrix L' and generates the sparse matrix S' by multiplying the sparse matrix S by the zero matrix. In other words, a weight coefficient of the low-rank matrix L is "1" and a weight coefficient of the sparse matrix S is "0." This is not limiting and any weight coefficient other than the above may be set. For example, the threshold processor 34 may generate the low-rank matrix L' by multiplying the low-rank matrix L by a matrix where a plurality of matrix elements is individually "1" or "0" or may generate the low-rank matrix L' by multiplying the sparse matrix S by a matrix where a plurality of matrix elements is individually in a range from "0" to "1." Likewise, the threshold processor 34 may generate the sparse matrix S' by multiplying the sparse matrix S by a matrix where a plurality of matrix elements is individually "1" or "0" or may generate the sparse matrix S' by multiplying the sparse matrix S by a matrix where a plurality of matrix elements is individually in a range from "0" to "1." The threshold processor 34 is able to use, for example, a weight coefficient to set a matrix to be multiplied by the low-rank matrix L and a matrix to be multiplied by the sparse matrix S.

In addition, for example, the threshold processor 34 may evaluate an autocorrelation in the time-axis direction in the low-rank matrix L, evaluate an autocorrelation in the time-axis direction in the sparse matrix S, and perform a process on the basis of the results of the evaluations. Specifically, the threshold processor 34 is able to set a weight coefficient on the basis of the results of the evaluations and use the weight coefficient to set a matrix to be multiplied by the low-rank matrix L and a matrix to be multiplied by the sparse matrix S.

[Modification Example 7]

In the above-described embodiment, the noise detector 12 calculates the time ratio RN of a period where a myoelectric noise is contained in the potential signal E1 to the entire period of the potential signal E1; however, this is not limiting. Alternatively, for example, the noise detector 12 may use a machine learning technology to output a score on the basis of the potential signal E1, the score indicating the degree of inclusion of a myoelectric noise in the potential signal E1.

[Modification Example 8]

In the above-described embodiment, the signal generator 31 generates the potential signal E31 by combining the potential signal E1 with the potential signal E2 supplied from the storage 14 on the time axis; however, this is not limiting. Alternatively, the signal generator 31 may combine the potential signal E1 and a portion of the potential signal E2 on the time axis. For example, the signal generator 31 is able to adjust a length of a portion of the potential signal E2 to be coupled to the potential signal E1 to cause the time ratio of a period when a myoelectric noise is contained to the combined entire period to reach a predetermined ratio.

[Modification Example 9]

In the above-described embodiment, the storage 14 stores the potential signal E2 during the period of the time length T but may store a plurality of previous potential signals E2. In this case, the signal generator 31 is able to generate the potential signal E31 by, for example, combining the potential signal E1 with one of the plurality of potential signals E2 stored in the storage 14 on the time axis. In this case, for example, the signal generator 31 may combine the potential signal E1 with the latest one of the plurality of potential signals E2 stored in the storage 14 or combine the potential signal E1 with, among the plurality of the potential signals E2 stored in the storage 14, one different from the latest potential signal E2. In addition, for example, the signal generator 31 may generate the potential signal E31 by generating an average signal of the plurality of potential signals E2 stored in the storage 14 and combining the potential signal E1 with the average signal on the time axis. In addition, for example, the signal generator 31 may generate the potential signal E31 by combining the potential signal E1 with the plurality of potential signals E2 supplied from the storage 14 on the time axis.

[Modification Example 10]

In the above-described embodiment, the storage 14 stores the potential signal E2; however, this is not limiting. A detailed description will be made below on a measurement device 1A according to the present modification example.

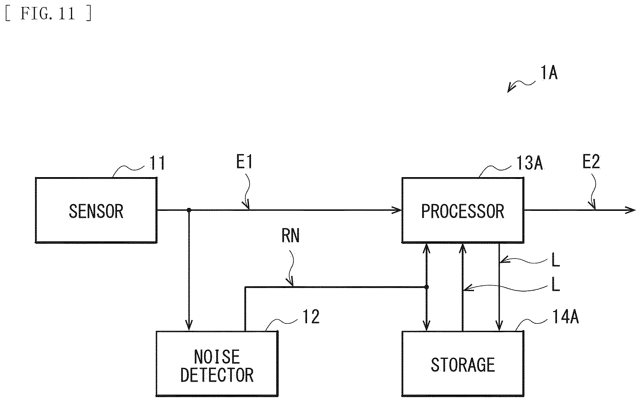

FIG. 11 illustrates a configuration example of the measurement device 1A. The measurement device 1A includes a processor 13A and a storage 14A.

The processor 13A is configured to generate a potential signal E2 by performing a noise reduction process on the basis of a potential signal E1 supplied from the sensor 11. The processor 13A performs the noise reduction process on the basis of the potential signal E1 in a case where the time ratio RN of the noise obtained by the noise detector 12 is equal to or less than the predetermined value TH. Meanwhile, in a case where the time ratio RN of the noise is greater than the predetermined value TH, the processor 13A performs the noise reduction process using a low-rank matrix L supplied from the storage 14 on the basis of the potential signal E1.

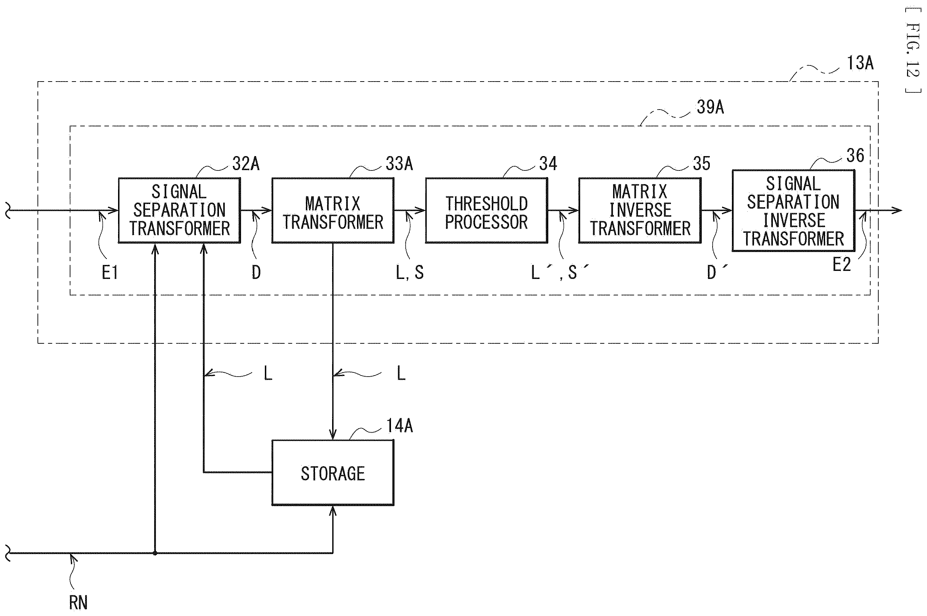

FIG. 12 illustrates a configuration example of the processor 13A. The processor 13A includes a noise reduction processor 39A. The noise reduction processor 39A includes a signal separation transformer 32A and a matrix transformer 33A.

The signal separation transformer 32A is configured to generate a matrix D by separating the potential signal E31 into signals with a plurality of frequency components. This operation is similar to the operation of the signal separation transformer 32 according to the above-described embodiment. Moreover, in a case where the time ratio RN of the noise obtained by the noise detector 12 is equal to or less than the predetermined value TH, the signal separation transformer 32A directly outputs the matrix D. Meanwhile, in a case where the time ratio RN of the noise obtained by the noise detector 12 is greater than the predetermined value TH, the signal separation transformer 32A updates the matrix D by combining the matrix D with the low-rank matrix L supplied from the storage 14A and outputs the updated matrix D. The signal separation transformer 32A is thus able to generate the matrix D similar to, for example, that in the case of the above-described embodiment (FIG. 10).

The matrix transformer 33A is configured to generate a low-rank matrix L and a sparse matrix S by applying a matrix separation process to the matrix D. This operation is similar to that of the matrix transformer 33 according to the above-described embodiment. Moreover, the matrix transformer 33A supplies the generated low-rank matrix L and sparse matrix S to the threshold processor 34 and supplies the low-rank matrix L to the storage 14A.

The storage 14A (FIG. 11) is configured to store the low-rank matrix L generated by the processor 13A. Specifically, in a case where the time ratio RN of the noise obtained by the noise detector 12 is equal to or less than the predetermined value TH, the storage 14A stores the low-rank matrix L generated by the processor 13A. Moreover, in a case where the time ratio RN of the noise is greater than the predetermined value TH, the storage 14A supplies the stored low-rank matrix L to the processor 13A.

Such a configuration also allows the measurement device 1A to operate similarly to the measurement device 1 according to the above-described embodiment.

[Other Modification Examples]

Two or more of these modification examples may be combined.

Hereinbefore, the present technology is described with reference to the embodiment and the several modification examples; however, the present technology is by no means limited to the embodiment and the like and a variety of modifications are possible.

For example, in the above-described embodiments, a brain wave is to be detected; however, this is not limiting and a variety of biological information is detectable.

It should be noted that the effects described herein are merely by way of example and not limiting and another effect may be produced.

It should be noted that the present technology may be implemented by the following configuration. The present technology with the following configuration makes it possible to enhance detection accuracy.

(1) A measurement device including:

a sensor configured to generate a first biological signal that is based on first biological information;
a processor configured to generate a second biological signal by performing a noise reduction process on the basis of the first biological signal; and
a storage configured to store a processing result of the noise reduction process, in which
the processor is configured to perform the noise reduction process using the processing result stored in the storage on the basis of the first biological signal.

(2) The measurement device according to (1), further including a noise detector configured to calculate a noise degree of inclusion, in the first biological signal, of a noise that is based on second biological information, in which the processor is configured to:

perform the noise reduction process using the processing result stored in the storage on the basis of the first biological signal in a case where the noise degree is greater than a predetermined value; and
perform the noise reduction process on the basis of the first biological signal in a case where the noise degree is smaller than the predetermined value.

(3) The measurement device according to (2), in which
during the noise reduction process, the processor is configured to:

generate a first matrix by separating the first biological signal into a plurality of signals;
perform a matrix separation process to separate the first matrix into a second matrix and a third matrix, the second matrix including a component less changeable in a time-axis direction, the third matrix including a component discrete in the time-axis direction; and
generate the second biological signal on the basis of the second matrix.

(4) The measurement device according to (3), in which, during the matrix separation process,
the processor is configured to generate a fourth matrix by performing an emphasis process to emphasize the third matrix and separate the first matrix into the second matrix and the third matrix to cause the second matrix and the fourth matrix to satisfy a predetermined condition.

(5) The measurement device according to (3) or (4), in which

the processing result stored in the storage includes the second biological signal generated through the noise reduction process in a past, and

in the case where the noise degree is greater than the predetermined value, the processor is configured to:

update the first biological signal by combining the first biological signal with the processing result stored in the storage; and perform the noise reduction process on the basis of the updated first biological signal.

(6) The measurement device according to (3) or (4), in which

the processing result stored in the storage includes the second matrix generated through the noise reduction process in a past, and

in the case where the noise degree is greater than the predetermined value, the processor is configured to:

update the first matrix by combining the first matrix with the processing result stored in the storage; and separate the updated first matrix into the second matrix and the third matrix.

(7) The measurement device according to any one of (2) to (6), in which
the storage is configured to store the processing result of the noise reduction process in the case where the noise degree is smaller than the predetermined value.

(8) The measurement device according to any one of (2) to (7), in which
the second biological information includes activity information regarding a muscle fiber.

(9) The measurement device according to any one of (1) to (8), in which
the first biological information includes brain wave information.

(10) A measurement method including:

generating a first biological signal that is based on first biological information;
generating a second biological signal by performing a noise reduction process on the basis of the first biological signal; and
storing a processing result of the noise reduction process, in which
the noise reduction process is performed using the stored processing result on the basis of the first biological signal.

This application claims priority to Japanese Patent Application No. 2021-151823 filed with the Japan Patent Office on September 17, 2021, the entire contents of which are incorporated herein by reference.

It should be understood by those skilled in the art that various modifications, combinations, sub-combinations, and alterations may occur depending on design requirements and other factors insofar as they are within the scope of the appended claims or the equivalents thereof.

## Claims

1. A measurement device comprising:

a sensor configured to generate a first biological signal that is based on first biological information;
a processor configured to generate a second biological signal by performing a noise reduction process on a basis of the first biological signal; and
a storage configured to store a processing result of the noise reduction process, wherein
the processor is configured to perform the noise reduction process using the processing result stored in the storage on the basis of the first biological signal.

2. The measurement device according to claim 1, further comprising a noise detector configured to calculate a noise degree of inclusion, in the first biological signal, of a noise that is based on second biological information, wherein
the processor is configured to:

perform the noise reduction process using the processing result stored in the storage on the basis of the first biological signal in a case where the noise degree is greater than a predetermined value; and
perform the noise reduction process on the basis of the first biological signal in a case where the noise degree is smaller than the predetermined value.

3. The measurement device according to claim 2, wherein
during the noise reduction process, the processor is configured to:

generate a first matrix by separating the first biological signal into a plurality of signals;

perform a matrix separation process to separate the first matrix into a second matrix and a third matrix, the second matrix including a component less changeable in a time-axis direction, the third matrix including a component discrete in the time-axis direction; and

generate the second biological signal on a basis of the second matrix.

4. The measurement device according to claim 3, wherein, during the matrix separation process, the processor is configured to generate a fourth matrix by performing an emphasis process to emphasize the third matrix and separate the first matrix into the second matrix and the third matrix to cause the second matrix and the fourth matrix to satisfy a predetermined condition.

5. The measurement device according to claim 3, wherein

the processing result stored in the storage comprises the second biological signal generated through the noise reduction process in a past, and

in the case where the noise degree is greater than the predetermined value, the processor is configured to:

update the first biological signal by combining the first biological signal with the processing result stored in the storage; and perform the noise reduction process on a basis of the updated first biological signal.

6. The measurement device according to claim 3, wherein

the processing result stored in the storage comprises the second matrix generated through the noise reduction process in a past, and in the case where the noise degree is greater than the predetermined value, the processor is configured to:

update the first matrix by combining the first matrix with the processing result stored in the storage; and separate the updated first matrix into the second matrix and the third matrix.

7. The measurement device according to claim 2, wherein

the storage is configured to store the processing result of the noise reduction process in the case where the noise degree is smaller than the predetermined value.

8. The measurement device according to claim 2, wherein

the second biological information comprises activity information regarding a muscle fiber.

9. The measurement device according to claim 1, wherein

the first biological information comprises brain wave information.

10. A measurement method comprising:

generating a first biological signal that is based on first biological information;

generating a second biological signal by performing a noise reduction process on a basis of the first biological signal; and

storing a processing result of the noise reduction process, wherein

the noise reduction process is performed using the stored processing result on the basis of the first biological signal.

[ FIG. 1 ]

[ FIG. 2 ]

[ FIG. 3A ]

[ FIG. 3B ]

[ FIG. 4 ]

13

39

31 SIGNAL GENERATOR — E1

32 SIGNAL SEPARATION TRANSFORMER — E31

D

33 MATRIX TRANSFORMER

L, S

34 THRESHOLD PROCESSOR

L', S'

35 MATRIX INVERSE TRANSFORMER

D'

36 SIGNAL SEPARATION INVERSE TRANSFORMER — E2

14 STORAGE

E2

RN

[ FIG. 5 ]

[ FIG. 6 ]

[ FIG. 7 ]

```
                      ┌─────────────┐
                      │    START    │
                      └──────┬──────┘
                             │                    S101
        ┌────────────────────┴────────────────────┐
        │ CALCULATE TIME RATIO RN OF               │
        │ PERIOD WHEN MYOELECTRIC NOISE            │
        │ IS CONTAINED IN POTENTIAL                │
        │ SIGNAL E1 TO ENTIRE PERIOD               │
        │ OF POTENTIAL SIGNAL E1                   │
        └────────────────────┬────────────────────┘
                             │
                             │            S102      N
               ◇─────────────────────────────◇──────────────────┐
                      RN ≤ TH?                                    │
               ◇─────────────────────────────◇                   │
                             │ Y                                  │
                             │        S103                        │              S105
        ┌────────────────────┴──────────────┐   ┌────────────────┴──────────────────┐
        │ GENERATE POTENTIAL SIGNAL E2       │   │ GENERATE POTENTIAL SIGNAL E31      │
        │ BY PERFORMING NOISE REDUCTION      │   │ BY COMBINING POTENTIAL SIGNAL      │
        │ PROCESS ON THE BASIS OF            │   │ E1 WITH POTENTIAL SIGNAL E2        │
        │ POTENTIAL SIGNAL E31               │   │ SUPPLIED FROM STORAGE 14 ON        │
        │ (POTENTIAL SIGNAL E1)              │   │ TIME AXIS                          │
        └────────────────────┬──────────────┘   └────────────────┬──────────────────┘
                             │        S104                        │              S106
        ┌────────────────────┴──────────────┐   ┌────────────────┴──────────────────┐
        │ STORE POTENTIAL SIGNAL E2          │   │ GENERATE POTENTIAL SIGNAL E2       │
        └────────────────────┬──────────────┘   │ BY PERFORMING NOISE REDUCTION      │
                             │                    │ PROCESS ON THE BASIS OF            │
                             │                    │ POTENTIAL SIGNAL E31               │
                             │                    └────────────────┬──────────────────┘
                             │                                     │              S107
                             │                    ┌────────────────┴──────────────────┐
                             │                    │ UPDATE POTENTIAL SIGNAL E2 BY      │
                             │                    │ REMOVING SIGNAL PORTION            │
                             │                    │ CORRESPONDING TO POTENTIAL         │
                             │                    │ SIGNAL E1 FROM POTENTIAL           │
                             │                    │ SIGNAL E2                          │
                             │                    └────────────────┬──────────────────┘
                             │◄───────────────────────────────────┘
                      ┌──────┴──────┐
                      │     END     │
                      └─────────────┘
```

17

[ FIG. 8 ]

[ FIG. 9 ]

[ FIG. 10 ]

[ FIG. 11 ]

~ 1A

SENSOR ~11    E1    PROCESSOR ~13A    E2

RN

NOISE DETECTOR ~12    STORAGE ~14A

L
L

[ FIG. 12 ]

| INTERNATIONAL SEARCH REPORT | International application No. |
|---|---|
| | **PCT/JP2022/010415** |

**A. CLASSIFICATION OF SUBJECT MATTER**

*A61B 5/31*(2021.01)i; *A61B 5/369*(2021.01)i
FI:    A61B5/31; A61B5/369

According to International Patent Classification (IPC) or to both national classification and IPC

**B. FIELDS SEARCHED**

Minimum documentation searched (classification system followed by classification symbols)

A61B5/31; A61B5/369

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2002-034944 A (RIKEN) 05 February 2002 (2002-02-05) paragraphs [0005]-[0013] | 1-4, 6-10 |
| A | | 5 |
| A | JP 2006-006665 A (OLYMPUS CORP.) 12 January 2006 (2006-01-12) paragraphs [0023]-[0044] | 5 |
| A | CN 106236080 A (HEFEI UNIVERSITY OF TECHNOLOGY) 21 December 2016 (2016-12-21) entire text, all drawings | 1-10 |
| A | US 2014/0107521 A1 (CASE WESTERN RESERVE UNIVERSITY) 17 April 2014 (2014-04-17) entire text, all drawings | 1-10 |
| A | WO 2008/084800 A1 (PANASONIC CORP.) 17 July 2008 (2008-07-17) entire text, all drawings | 1-10 |

☐ Further documents are listed in the continuation of Box C.       ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 May 2022** | **24 May 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**
Information on patent family members

International application No.

**PCT/JP2022/010415**

| Patent document cited in search report | | | Publication date (day/month/year) | Patent family member(s) | Publication date (day/month/year) |
|---|---|---|---|---|---|
| JP | 2002-034944 | A | 05 February 2002 | (Family: none) | |
| JP | 2006-006665 | A | 12 January 2006 | (Family: none) | |
| CN | 106236080 | A | 21 December 2016 | (Family: none) | |
| US | 2014/0107521 | A1 | 17 April 2014 | (Family: none) | |
| WO | 2008/084800 | A1 | 17 July 2008 | US  2010/0197262  A1 entire text, all drawings CN  101542947  A | |

Form PCT/ISA/210 (patent family annex) (January 2015)

23

**EP 4 385 412 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001000407 A
- JP 2021151823 A